(19) Europäisches Patentamt
European Patent Office
Office européen des brevets

(11) Veröffentlichungsnummer: **0 079 623**
**B1**

(12) # EUROPÄISCHE PATENTSCHRIFT

(45) Veröffentlichungstag der Patentschrift: **21.08.85**

(51) Int. Cl.⁴: **C 07 D 207/337**

(21) Anmeldenummer: **82200311.7**

(22) Anmeldetag: **10.03.82**

(54) **Verbessertes Verfahren zur Herstellung von 1H-Pyrrol-2-essigsäureestern.**

(30) Priorität: **17.11.81 DE 3145510**

(43) Veröffentlichungstag der Anmeldung: **25.05.83 Patentblatt 83/21**

(45) Bekanntmachung des Hinweises auf die Patenterteilung: **21.08.85 Patentblatt 85/34**

(84) Benannte Vertragsstaaten: **AT BE CH DE FR GB IT LI LU NL SE**

(56) Entgegenhaltungen:
**DE - B - 1 301 313**

**TETRAHEDRON LETTERS, Nr. 27, 1975, Seiten 2303-2306, Pergamon Press, G.B., K. HOLMBERG et al.: "Methylene diesters of carboxylic acids from dichloromethane"**

(73) Patentinhaber: **Rütgerswerke Aktiengesellschaft, Mainzer Landstrasse 217, D-6000 Frankfurt a.Main 1 (DE)**

(72) Erfinder: **Orth, Winfried, Dr., Am Schachtelgraben 28, D-6733 Hassloch/Pfalz (DE)**
Erfinder: **Fickert, Werner, Dr., Stockacher Strasse 14, D-6800 Mannheim 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 079 623 B1

BUNDESDRUCKEREI BERLIN

**Beschreibung**

Die Erfindung betrifft ein neues Verfahren zur Herstellung von Pyrrol-2-essigsäureestern der allgemeinen Formel

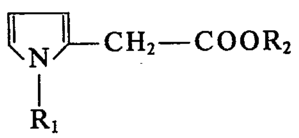

in der $R_1$ ein Wasserstoffatom oder eine Alkylgruppe mit 1 bis 4 Kohlenstoffatomen und $R_2$ eine Aralkyl- oder Alkylgruppe mit 1 bis 4 Kohlenstoffatomen bedeutet, aus den entsprechenden Pyrrol-2-acetonitrilen und/oder -acetamiden.

Diese Pyrrol-2-essigsäureester sind Ausgangsprodukte für die Herstellung von bekannten Phenothiazinderivaten, die als Antihistaminica und Spasmolytica Verwendung finden.

Aus der DE-PS 1 301 313 ist ein Verfahren zur Herstellung von Pyrrol-2-essigsäureestern aus den Nitrilen und/oder Amiden bekannt, bei dem die Nitrile oder Amide in Gegenwart von niedrigen Glykolen und deren Äthern mit Alkalilaugen bei erhöhten Temperaturen behandelt werden. Danach wird das Wasser aus dem Reaktionsgemisch abdestilliert und die erhaltenen Alkalisalze der Pyrrol-2-esigsäure mit Alkyl- oder Aralkylhalogeniden in Gegenwart von Ketonen als Lösungsmittel verestert, ohne besonders isoliert zu werden.

Obwohl diese Herstellungsmethode ein Eintopfverfahren darstellt, ist sie insofern aufwendig, als das zum 1. Reaktionsschritt notwendige Wasser abdestilliert werden muß und daß ein Lösungsmittelgemisch entsteht, das gesondert aufgearbeitet werden muß. Außerdem werden, bedingt durch mangelnde Isomerenreinheit der Ausgangsnitrile bzw. -amide nach dem Verfahren Pyrrol-2-essigsäureester mit einem relativ hohen Gehalt an isomeren Beiprodukten erhalten.

Aufgabe der Erfindung war es daher, ein Verfahren zu entwickeln, mit dem man in einem einfach durchzuführenden Eintopfverfahren ohne weiteren Aufwand 1H-Pyrrol-2-essigsäureester aus Pyrrol-2-acetonitrilen und/oder -acetamiden herstellen kann.

Des weiteren bestand die Aufgabe, 1H-Pyrrol-2-essigsäureester in einfacher Weise isomerenfrei herzustellen.

Die Aufgabe wird dadurch gelöst, daß die Verseifung der Nitrile und/oder Amide mit Laugen ohne Verwendung eines Lösungsvermittlers durchgeführt wird und die erhaltenen Salze der Pyrrol-2-essigsäuren ohne Isolierung mit Alkylierungs- oder Aralkylierungsmitteln in einem nicht wasserlöslichen organischen Lösungsmittel mit Hilfe eines Phasen-Transfer-Katalysators umgesetzt werden.

Die isomerenfreien 1H-Pyrrol-2-essigsäureester werden dadurch erhalten, daß die Verseifung der Nitrile und/oder Amide ohne Verwendung eines Lösungsvermittlers durch allmähliche Zugabe von Lauge durchgeführt wird und die erhaltenen Salze der Pyrrol-2-essigsäuren ohne Isolierung mit Alkylierungs- oder Aralkylierungsmitteln in einem nicht wasserlöslichen organischen Lösungsmittel mit Hilfe eines Phasen-Transfer-Katalysators umgesetzt werden.

Es wurde gefunden, daß sich — entgegen der Aussage der DE-PS 1 301 313 — die Verseifung der Pyrrolacetonitrile bzw. -acetamide auch ohne Zugabe von organischen Lösungsmitteln, die als Lösungsvermittler wirken, durchführen läßt, wenn man diese Nitrile bzw. Amide mit wäßrigen Laugenlösungen in der Wärme behandelt. Die Verseifungsreaktion erfolgt an der Grenzfläche zwischen organischer und wäßriger Phase und die sich bildenden Salze der Pyrrol-2-essigsäuren lösen sich in der wäßrigen Phase.

Als Pyrrol-2-acetonitrile können sowohl Reinprodukte als auch die Rohnitrile zur Umsetzung eingesetzt werden, wie sie bei Durchführung der entsprechenden Herstellungsverfahren, z. B. nach DE-PS 1 301 312, hergestellt werden. Diese Rohnitrile enthalten außer Nitrilen stets wechselnde Mengen an Pyrrol-2-acetamiden. Da aber die Pyrrol-2-acetamide unter den erfindungsgemäßen Bedingungen auch zu den Alkalisalzen der Pyrrol-2-essigsäuren umgesetzt werden, und somit auch reine Pyrrol-2-acetamide eingesetzt werden können, stört ihre Anwesenheit in den Rohnitrilen nicht.

Als Laugen zur Durchführung des erfindungsgemäßen Verfahrens eignen sich wäßrige Lösungen der Alkalihydroxide oder der Hydroxide der Erdalkalimetalle Calcium, Strontium und Barium. Die Menge der Hydroxide muß dabei mindestens der stöchiometrischen Menge der eingesetzten Nitrile und/oder Amide entsprechen. Zweckmäßigerweise aber verwendet man einen Überschuß an Lauge von 30—100% der theoretisch notwendigen Menge.

Die Verseifung kann so durchgeführt werden, daß die gesamte Lauge auf einmal zugegeben und mit dem Nitril und/oder Amid in der Wärme umgesetzt wird. Es wurde aber gefunden, daß man bei allmählicher Zugabe von Lauge zu einer vorgelegten Pyrrol-2-acetonitril- und/oder -amidmenge isomerenfreie Salze der Pyrrol-2-essigsäure herstellen kann, aus denen man dann auch bei der Weiterverarbeitung ohne zusätzlichen Aufwand isomerenfreie Pyrrol-2-essigsäureester gewinnen kann.

Die Ursache für die Isomeren liegt bereits bei der Herstellung des Pyrrol-2-acetonitrils, bei der als Nebenprodukte noch das entsprechende 5-Methyl-1H-pyrrol-2-carbonitril gebildet wird, das bei der

Verseifung analog dem -acetonitril reagiert unter Bildung des entsprechenden isomeren 5-Methyl-1H-pyrrol-carbonsäureesters. Es wurde gefunden, daß die Reaktivitäten der jeweiligen isomeren Nitrile bzw. der jeweiligen isomeren Amide bei der Verseifung unterschiedlich sind, so daß man bei allmählicher Zugabe von Lauge zur Reaktionslösung die Pyrrol-2-acetonitrile zu den entsprechenden Carbonsäuren verseifen kann, während die 5-Methyl-2-carbonitrile unter diesen Bedingungen nur zu den entsprechenden Amiden hydrolysieren.

Der bei der Verseifungsreaktion frei werdende Ammoniak wird durch Kochen am Rückfluß ausgetrieben. Durch Zugabe von Alkalihydrogencarbonat wird ein pH-Wert von etwa 11 eingestellt, der für die weitere Umsetzung günstig ist.

Zur Weiterverarbeitung werden die Salze der Pyrrol-2-essigsäuren mit Alkylierungs- oder Aralkylierungsmitteln, wie z. B. mit Alkylhalogeniden, -sulfaten oder Aralkylhalogeniden in der Wärme umgesetzt, wobei die Reaktionstemperatur von der Reaktivität der Alkylierungs- oder Aralkylierungsmittel abhängig ist. Umsetzungen mit Alkylierungsmitteln, die unter den Reaktionsbedingungen gasförmig sind, wie z. B. Methyl-, Äthyl- oder Isopropylbromid, werden unter leichtem Überdruck durchgeführt, der ein Verdampfen des Alkylierungsmittels verhindert.

Während die Salze der Pyrrol-2-essigsäuren in der wäßrigen Phase gelöst sind, werden die Alkylierungs- oder Aralkylierungsmittel in einem mit Wasser nicht mischbaren organischen Lösungsmittel zugegeben. Die Veresterungsreaktion wird mit Phasen-Transfer-Katalysatoren katalysiert. Als Phasen-Transfer-Katalysatoren können die üblicherweise für diesen Zweck verwendeten Substanzen wie Phosphoniumsalze, bevorzugt aber Ammoniumbasen, wie z. B. Tetrabutylammonium-hydrogensulfat eingesetzt werden. Sie bedingen eine optimale, bis zu 100prozentige Ausbeute.

Überraschenderweise wurde festgestellt, daß anstelle von Ammoniumbasen auch Amine dann als Katalysator zu reagieren vermögen, wenn diese sich mit dem angebotenen Alkylierungsmittel zu einer quaternären Ammoniumbase umzusetzen vermögen. Wie die Beispiele zeigen, reicht es unter diesen Voraussetzungen sogar, nur ein primäres Amin einzusetzen. Die üblicherweise verwendeten Katalysatormengen liegen zwischen 0,25 und 5 g pro Mol des umzusetzenden Salzes der Pyrrol-2-essigsäure. Bei geringeren Katalysatormengen verläuft die Umsetzung zu langsam. Die Verwendung größerer Katalysatormengen ist unwirtschaftlich, besonders wenn teure Ammoniumbasen eingesetzt werden.

## Beispiele

### Beispiel 1

Zu einer Mischung aus 1200 g (7,7 Mol) 1-Methyl-1H-pyrrol-2-acetonitril (Rohnitril) und 920 ml Wasser werden unter Rühren in einer Stunde bei einer Sumpftemperatur von 98—100° C 920 g (11,5 Mol) 50proz. Natronlauge getropft. Anschließend wird das Verseifungsgemisch noch weitere 2,5 Stunden bei der selben Temperatur nachgerührt. Dann werden 462 g (5,5 Mol) gepulvertes Natriumbicarbonat zugesetzt und die Reaktionsmischung noch 30 Min. lang zum Rückfluß erhitzt. Danach werden 4 l Toluol und 10 g Tetrabutylammonium-hydrogensulfat zugegeben und während 3 Stunden bei einer Sumpftemperatur um 70° C 1607 g (12,5 Mol) Dimethylsulfat gleichmäßig zugetropft, gefolgt von einer vierstündigen Nachreaktionszeit bei der gleichen Temperatur. Die Mischung wird dann abgekühlt, die wäßrige Phase abgetrennt und die organische unter schwachem Vakuum eingeengt. Vom Destillationsrückstand wird schließlich im Wasserstrahlvakuum der gebildete Ester unter Verwendung einer kurzen Kolonne überdestilliert. Nach Abnahme einer geringen Menge eines Vorlaufs von 95—118° C/19 mbar geht bei 118—120° C/19 mbar als Hauptlauf 98—99%iger 1-Methyl-1H-pyrrol-2-essigsäuremethylester in einer Ausbeute von 98—100%, bezogen auf die theoretische Esterausbeute, über. Als Destillationsrückstand verbleibt das 1,5-Dimethyl-1H-pyrrol-2-carboxamid.

### Beispiel 2

Zu einer gerührten Mischung als 400 g (2,57 Mol) 1-Methyl-1H-pyrrol-2-acetonitril (Rohnitril) und 310 ml Wasser werden in einer Stunde bei einer Sumpftemperatur von 98—100° C 307 g (3,8 Mol) 50proz. Natronlauge getropft. Anschließend wird das Verseifungsgemisch noch weitere 2,5 Stunden bei der selben Temperatur nachgerührt. Dann werden 154 g (2,19 Mol) gepulvertes Natriumbicarbonat zugesetzt und die Reaktionsmischung noch 30 Min. lang zum Rückfluß erhitzt. Danach werden 1,3 l Toluol und 10 g Trioctylamin zugegeben und während 3 Stunden bei einer Sumpftemperatur um 70° C 536 g (4,24 Mol) Dimethylsulfat gleichmäßig zugetropft, gefolgt von einer vierstündigen Nachreaktionszeit bei der gleichen Temperatur. Die Mischung wird dann abgekühlt, die wäßrige Phase abgetrennt und die organische unter schwachem Vakuum eingeengt. Vom Destillationsrückstand wird schließlich im Wasserstrahlvakuum der gebildete Ester unter Verwendung einer kurzen Kolonne überdestilliert. Nach Abnahme einer geringen Menge eines Vorlaufs von 95—118° C/19 mbar geht bei 118—120° C/19 mbar als Hauptlauf 97—98%iger 1-Methyl-1H-pyrrol-2-essigsäuremethylester in einer Ausbeute von 97—99% der Theorie über. Als Destillationsrückstand verbleibt das 1,5-Dimethyl-1H-pyrrol-2-carboxamid.

Beispiele 3—10

Analog zu Beispiel 2 werden jeweils 400 g 1-Methyl-1H-pyrrol-2-acetonitril (Rohnitril) verseift und die entstandenen Salze der 1-Methylpyrrol-2-essigsäure verestert. Variiert werden dabei die verwendete Lauge, die Alkylierungs- bzw. Aralkylierungsmittel und die Phasen-Transfer-Katalysatoren. Bei der Veresterung mit den Alkylierungsmitteln Methyl-, Äthyl- u. Isopropylbromid wurde unter einem Überdruck bis etwa 1,5 bar gearbeitet. Die folgende Aufstellung zeigt die erhaltenen Ergebnisse.

| Beispiel | Lauge | Alkylierungs- bzw. Aralkylierungsmittel | Katalysator | Temperatur bei Veresterung [°C]/Überdruck [bar] | Ausbeute [% d. Theorie] |
|---|---|---|---|---|---|
| 3 | NaOH | Methylbromid | Tetrabutylammonium-hydrogensulfat | 90°C/1,5 | 96,8 |
| 4 | KOH | Isopropylbromid | Tetrabutylammonium-hydrogensulfat | 90°C/0,8 | 97,5 |
| 5 | NaOH | n-Butylbromid | Tripropylamin | 100°C/0,0 | 95,2 |
| 6 | NaOH | Dimethylsulfat | Diäthylamin | 70°C/0,0 | 97,8 |
| 7 | NaOH | Äthylbromid | Monooctylamin | 90°C/0,9 | 96,1 |
| 8 | NaOH | Isopropylbromid | Dicyclohexylamin | 90°C/0,8 | 95,6 |
| 9 | NaOH | Benzylchlorid | Triäthylamin | 100°C/0,0 | 95,1 |
| 10 | $Ba(OH)_2$ | n-Butylbromid | Tetrabutylamin | 100°C/0,0 | 94,1 |

**Patentansprüche**

1. Verfahren zur Herstellung von Pyrrol-2-essigsäureestern der allgemeinen Formel

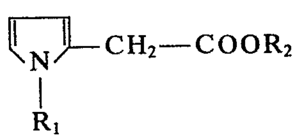

in der $R_1$ ein Wasserstoffatom oder eine Alkylgruppe mit 1 bis 4 Kohlenstoffatomen und $R_2$ eine Aralkyl- oder Alkylgruppe mit 1 bis 4 Kohlenstoffatomen bedeutet, durch Verseifung der entsprechenden Pyrrol-2-acetonitrile und/oder -acetamide mit Laugen und anschließende Alkylierung ohne Zwischenisolierung der Salze, dadurch gekennzeichnet, daß die Verseifung der Nitrile und/oder Amide ohne Verwendung eines Lösungsvermittlers durchgeführt wird und die erhaltenen Salze der Pyrrol-2-essigsäuren mit Alkylierungs- oder Aralkylierungsmitteln in einem nicht wasserlöslichen organischen Lösungsmittel mit Hilfe eines Phasen-Transfer-Katalysators umgesetzt werden, wobei als Phasen-Transfer-Katalysator ein Phosphoniumsalz, quaternäres Ammoniumsalz oder ein primäres, sekundäres oder tertiäres Amin, das sich mit dem angebotenen Alkylierungsmittel zu einem quaternären Ammoniumsalz umsetzt, verwendet werden.

2. Verfahren zur Herstellung von isomerenfreien Pyrrol-2-essigsäureestern der allgemeinen Formel

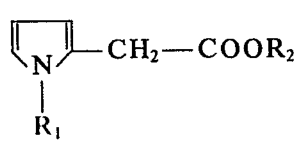

in der $R_1$ ein Wasserstoffatom oder eine Alkylgruppe mit 1 bis 4 Kohlenstoffatomen und $R_2$ eine Aralkyl- oder Alkylgruppe mit 1 bis 4 Kohlenstoffatomen bedeutet, durch Verseifung der entsprechenden Pyrrol-2-actonitrile und/oder -acetamide, mit Laugen und anschließende Alkylierung ohne Zwischenisolierung der Salze, dadurch gekennzeichnet, daß die Verseifung der Nitrile und/oder Amide ohne Verwendung eines Lösungsvermittlers durch allmähliche Zugabe von Lauge durchgeführt wird und die erhaltenen Salze der Pyrrol-2-essigsäuren mit Alkylierungs- oder Aralkylierungsmitteln in einem nicht wasserlöslichen organischen Lösungsmittel mit Hilfe eines Phasen-Transfer-Katalysators umgesetzt werden, wobei als Phasen-Transfer-Katalysator Phosphoniumsalz, quaternäres Ammoniumsalz oder ein primäres, sekundäres oder tertiäres Amin, das sich mit dem angebotenen Alkylierungsmittel zu einem quaternären Ammoniumsalz umsetzt, verwendet werden.

3. Verfahren nach Anspruch 1 oder 2, dadurch gekennzeichnet, daß als Lauge eine wäßrige Lösung eines Alkalihydroxids verwendet wird.

4. Verfahren nach Anspruch 1 oder 2, dadurch gekennzeichnet, daß als Lauge eine wäßrige Lösung eines Hydroxids des Calciums, Strontiums oder Bariums verwendet wird.

5. Verfahren nach einem oder mehreren der Ansprüche 1 bis 4, dadurch gekennzeichnet, daß als Alkylierungsmittel Alkylbromide verwendet werden.

6. Verfahren nach einem oder mehreren der Ansprüche 1 bis 4, dadurch gekennzeichnet, daß als Alkylierungsmittel Alkylsulfate verwendet werden.

7. Verfahren nach einem oder mehreren der Ansprüche 1 bis 6, dadurch gekennzeichnet, daß als Phasen-Transfer-Katalysator Tetrabutylammoniumhydrogensulfat verwendet wird.

## Claims

1. A process for the preparation of esters of 1H-pyrrol-2-acetic acids of the formula

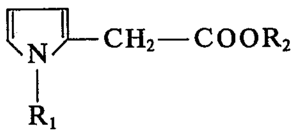

wherein $R_1$ is selected from the group consisting of hydrogen and alkyl of 1 to 4 carbon atoms and $R_2$ is selected from the group consisting of alkyl of 1 to 4 carbon atoms and aralkyl of 1 to 4 alkyl carbon atoms comprising saponifying the corresponding pyrrol-acetonitriles and/or acetamides with bases and subsequent alkylation without intermediate isolation of the salts, characterized in that the saponification of the nitriles and/or amides is made without the use of a solubilizer and the obtained salts of the pyrrol-2-acetic acid are reacted with alkylating or aralkylating agents in a water insoluble organic solvent under the aid of a phase transfer catalyst whereas as phase transfer catalyst a phosphonium salt, a quaternary ammonium salt or a primary, secundary or tertiary amine reacting with the alkylating agent to a quaternary ammonium salt can be used.

2. A process for the preparation of esters of 1H-pyrrol-2-acetic acids, free of isomers, of the formula

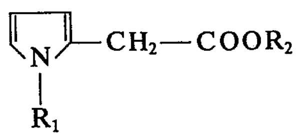

wherein $R_1$ is selected from the group consisting of hydrogen and alkyl of 1 to 4 carbon atoms and $R_2$ is selected from the group consisting of alkyl of 1 to 4 carbon atoms and aralkyl of 1 to 4 alkyl carbon atoms comprising saponifying the corresponding pyrrol-2-acetonitrile and/or -acetamide with bases and subsequent alkylation without intermediate isolation of the salts, characterized in that the saponification of the nitriles and/or amides is made without the use of a solubilizer by gradual addition of base and the obtained salts of the pyrrol-2-acetic acid are reacted with alkylating or aralkylating agents in a water insoluble, organic solvent under the aid of a phase transfer catalyst whereas as phase transfer catalyst a phosphonium salt, a quaternary ammonium salt or a primary, secundary or tertiary amine reacting with the alkylating agent to a quaternary ammonium salt can be used.

3. The process of claim 1 or 2 wherein the base is an aqueous alkali metal hydroxide solution.

4. The process of claim 1 or 2 wherein the base is an aqueous solution of a hydroxide of calcium, strontium or barium.

5. The process of one or several of the claims 1 to 4 wherein the alkylating agent is an alkyl bromide.

6. The process of one or several of the claims 1 to 4 wherein the alkylating agent is an alkyl sulfate.

7. The process of one or several of the claims 1 to 6 wherein the phase transfer catalyst is tetrabutyl ammonium sulfate.

**Revendications**

1. Procédé pour la préparation d'esters de l'acide pyrrol-2-acétique de formule générale:

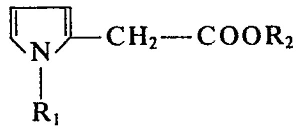

dans laquelle $R_1$ représente un atome d'hydrogène ou un groupe alcoyle ayant de 1 à 4 atomes de carbone et $R_2$ un groupe aralcoyle ou alcoyle ayant de 1 à 4 atomes de carbone, par saponification des pyrrol-2-acétonitriles et/ou -acétamides correspondants à l'aide de lessive et avec alcoylation subséquente sans isolement intermédiaire des sels, caractérisé par le fait que la saponification des nitriles et/ou amides est effectuée sans mise en oeuvre d'un agent de solubilisation et que les sels des acides pyrrol-2-acétiques obtenus sont mis à réagir avec des agents d'alcoylation ou d'aralcoylation au sein d'un solvant organique non hydrosoluble à l'aide d'un catalyseur de transfert de phase, le catalyseur de transfert de phase mis en oeuvre étant un sel de phosphonium, un sel d'ammonium quaternaire ou une amine primaire, secondaire ou tertiaire qui réagit avec l'agent d'alcoylation offert pour former un sel d'ammonium quaternaire.

2. Procédé pour la préparation d'esters de l'acide pyrrol-2-acétique exempts d'isomères de formule générale:

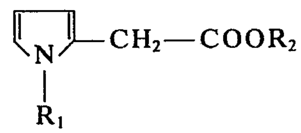

dans laquelle $R_1$ représente un atome d'hydrogène ou un groupe alcoyle ayant de 1 à 4 atomes de carbone et $R_2$ un groupe aralcoyle ou alcoyle ayant de 1 à 4 atomes de carbone, par saponification des pyrrol-2-acétonitriles et/ou -acétamides correspondants à l'aide de lessive et avec alcoylation subséquente sans isolement intermédiaire des sels, caractérisé par le fait que la saponification des nitriles et/ou amides est effectuée sans mise en oeuvre d'un agent de solubilisation et que les sels des acides pyrrol-2-acétiques obtenus sont mis à réagir avec des agents d'alcoylation ou d'aralcoylation au sein d'un solvant organique non hydrosoluble à l'aide d'un catalyseur de transfert de phase, le catalyseur de transfert de phase mis en oeuvre étant un sel de phosphonium, un sel d'ammonium quaternaire ou une amine primaire, secondaire ou tertiaire qui réagit avec l'agent d'alcoylation offert pour former un sel d'ammonium quaternaire.

3. Procédé selon la revendication 1 ou 2, caractérisé par le fait qu'uen tant que lessive on utilise une solution aqueuse d'un hydroxyde alcalin.

4. Procédé selon la revendication 1 ou 2, caractérisé par le fait qu'en tant que lessive on utilise une solution aqueuse d'un hydroxyde du calcium, strontium ou baryum.

5. Procédé selon l'une ou plusieurs des revendications 1 à 4, caractérisé par le fait qu'en tant qu'agents d'alcoylation on utilise des bromures d'alcoyle.

6. Procédé selon l'une ou plusieurs des revendications 1 à 4, caractérisé par le fait qu'en tant qu'agents d'alcoylation on utilise des sulfates d'alcoyle.

7. Procédé selon l'une ou plusieurs des revendications 1 à 6, caractérisé par le fait qu'en tant que catalyseur de transfert de phase, on utilise de l'hydrogénosulfate de tétrabutylammonium.